# EUROPEAN PATENT SPECIFICATION

(11) **EP 0 429 157 B1**
(45) Date of publication and mention of the grant of the patent: **22.03.1995**
(21) Application number: 90300697.1
(22) Date of filing: 23.01.1990
(51) Int. Cl.: C07C 59/265, A61K 31/19

(54) **Dietary supplementation with potassium magnesium citrate**
Magnesiumcitrat als Diätzusatzmittel
Supplémentation diététique pour citrate de magnésium

(30) Priority: 16.11.1989 US 437938
(43) Date of publication of application: 29.05.1991
(73) Proprietor: MISSION PHARMACAL COMPANY, INC., San Antonio, Texas 78296 (US); THE UNIVERSITY OF TEXAS SYSTEM, Austin, Texas 78701-2981 (US)
(72) Inventor: Walsdorf, Neill B., San Antonio Texas 78230 (US); Alexandrides, George, San Antonio Texas 78231 (US); Pak, Charles Y.C., Dallas Texas 75230 (US)
(74) Representative: Colgan, Stephen James

(56) References cited:
- DE-A- 2 252 665
- DE-C- 968 843
- US-A- 4 895 980

## Description

This invention relates to the use of potassium magnesium citrate for increasing urinary pH, potassium, magnesium and citrate through the oral administration of potassium magnesium citrate. Another aspect of the invention relates to a the use of this compound for decreasing urinary calcium and oxalate

### Background Of Invention

The use of magnesium salts for dietary supplementation is well known. Unfortunately, the beneficial effects derived from dietary magnesium supplementation have too often been achieved at the expense of other undesirable side effects such as acute diarrhea. Another disadvantage of the commercially available dietary magnesium supplements has been the relatively large tablet size required to obtain the desired magnesium dosage.

The use of potassium supplements such as potassium chloride for the treatment of patients with hypokalemia is also well known. Here again, however, problems have been encountered with associated side effects such as arrhythmia and diarrhea.

Recently, it has been learned that some of these undesirable side effects can be better controlled by administering potassium in combination with magnesium citrate. Nevertheless, due to the relatively low densities of the commercially available magnesium citrate preparations, the large tablet sizes required to obtain a desirable dosage remain a problem.

It is known that thiazides probably represent the most popular treatment regimen for hypercalciuric nephrolithiasis. They have even been advocated for the management of calcium nephrolithiasis associated with normocalciuria.

However, there are certain problems with thiazide therapy which may limit its utility in nephrolithiasis. First, thiazides may cause hypokalemia and magnesium depletion by provoking renal loss of potassium and magnesium. Particularly common in older subjects, this complication may cause muscle weakness, cramping and serious cardiac arrhythmias.

Second, the induced potassium and magnesium loss may impair renal excretion of citrate, a recognized inhibitor of the crystallization of calcium salts. The resulting reduction in inhibitor activity may cause a relapse in stone formation during thiazide therapy, by opposing the hypocalciuric action of thiazide. Despite numerous reports of the utility of thiazide in the control of hypercalciuric nephrolithiasis, experience with the use of thiazide alone has not been entirely satisfactory, with a relapse rate of 37.5 - 57.1%.

The above problems may be partly overcome by potassium citrate. It has previously been shown that potassium citrate averts the development of hypokalemia in hypercalciuric patients with nephrolithiasis treated with thiazide, augments citrate excretion and prevents recurrent stone formation in patients who have relapsed on thiazide therapy.

However, potassium citrate therapy does not avert thiazide-induced magnesium depletion. This need theoretically may be met by magnesium citrate. Magnesium citrate has been shown to be more soluble and absorbable than magnesium oxide. Unfortunately, magnesium citrate was also shown to have an equivalent magnesiuric and citraturic effect as magnesium oxide, when magnesium salts were provided in small divided doses.

Another problem with potassium citrate is poor patient compliance due to the small amount of potassium citrate (5 meq) contained in each tablet. Thus a patient maintained on an average dose of Urocit-K (50 meq/day) needs to take 10 tablets of this medication per day.

A dual mineral salt, potassium magnesium citrate (K₄Mg(C₆H₅O₇)₂) and its use as a dietary supplement for potassium and magnesium is known from EP-A-380 829 (cited under Article 54(3) and (4).

DE-A-2 252 665 describes compositions for the control of hypercalciuric nephrolithiasis containing potassium citrate and magnesium citrate. DE-A-968 843 describes the use of a complex magnesium alkali metal salt of citric acid in the treatment of the nervous system.

According to the present invention there is provided the use of potassium magnesium citrate in a single salt in the manufacture of an orally administrable medicament for increasing urinary citrate of magnesium excretion or for decreasing the urinary calcium or oxalate of an individual.

Potassium magnesium citrate may be used to ameliorate the hypokalemia and/or calcium deficiency induced when patients are treated with thiazide. The invention therefore also provides a product containing potassium magnesium citrate and a thiazide for their concomitant sequential use in therapy.

Details of the manufacture of calcium magnesium citrate may be obtained from EP-A-380 829.

Preferred features of the invention will now be described, by way of example, with reference to the following drawings and Examples. In the drawings:
Figure 1 is a graph demonstrating the comparative effects of potassium magnesium citrate and potassium citrate on urinary potassium;
Figure 2 is a graph demonstrating the comparative effects of potassium magnesium citrate and magnesium citrate on urinary magnesium; and
Figure 3 is a graph demonstrating the comparative effects of potassium magnesium citrate, magnesium citrate and potassium citrate on urinary citrate.

The benefits available to patients through administration of tetrapotassium monomagnesium dicitrate as disclosed herein are believed attributable to its unexpected desirable formulation characteristic. Thus, a single tablet of potassium magnesium citrate contains 7 meq potassium, 3.5 meq magnesium and 10.5 meq citrate. In contrast, the currently available formulation of potassium citrate (Urocit-K) of the same size as potassium magnesium citrate has only 5 meq potassium (29% less), 5 meq citrate (52% less) and no magnesium. Consider a patient taking a typical dose of potassium citrate 50 meq/day. The patient would need to take 10 tablets in order to provide 50 meq potassium and 50 meq citrate. In contrast, the patient would require only 7 tablets of potassium magnesium citrate in order to provide an equivalent amount of potassium (49 meq), more citrate (73.5 meq) as well as magnesium (24.5 meq).

The value of the tetrapotassium monomagnesium dicitrate composition disclosed herein as a dietary supplement is also enhanced by its excellent solubility. Four tablets of potassium magnesium citrate (containing 28 meq K, 14 meq Mg and 42 meq citrate, representing a high single dose) were found to be completely soluble in 300 ml water (without HC1) at 37 degrees C. after 15 min. incubation.

### Example 1

A study was done to demonstrate the excellent bioavailability of potassium, magnesium and citrate when administered orally in the form of tetrapotassium monomagnesium dicitrate. The study was performed with three normal subjects. Qualitatively similar findings were observed. The results in one subject are detailed below:

The subject underwent three phases of study. During one phase, the patient took potassium citrate (10 tablets Urocit-K or 50 meq potassium and citrate), at another time magnesium citrate (2.5 tablets containing 25 meq magnesium and citrate), and at a third time potassium magnesium citrate (7 tablets containing 49 meq K, 73.5 meq citrate and 24.5 meq Mg). Urine was collected at frequent intervals for 24 hours after oral ingestion of each salt (at 8 a.m.).

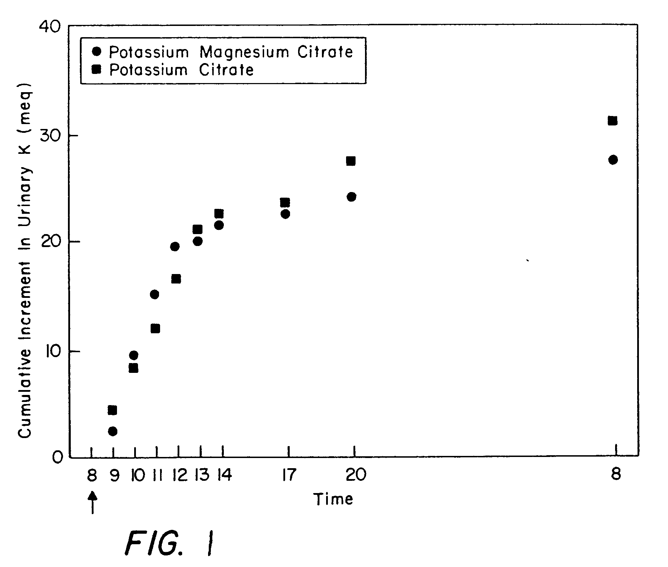

Potassium bioavailability from potassium magnesium citrate was compared with that of potassium citrate. At each time period, the difference in urinary potassium following ingestion of potassium magnesium citrate (containing 49 meq potassium) or potassium citrate (containing 50 meq potassium) from that obtained at a corresponding time period following taking magnesium citrate (no potassium; therefore serving as control). The cumulative increment in urinary potassium (indicative of potassium bioavailability) is shown in Figure 1. As shown, potassium bioavailability was equivalent between the two preparations (potassium magnesium citrate and potassium citrate).

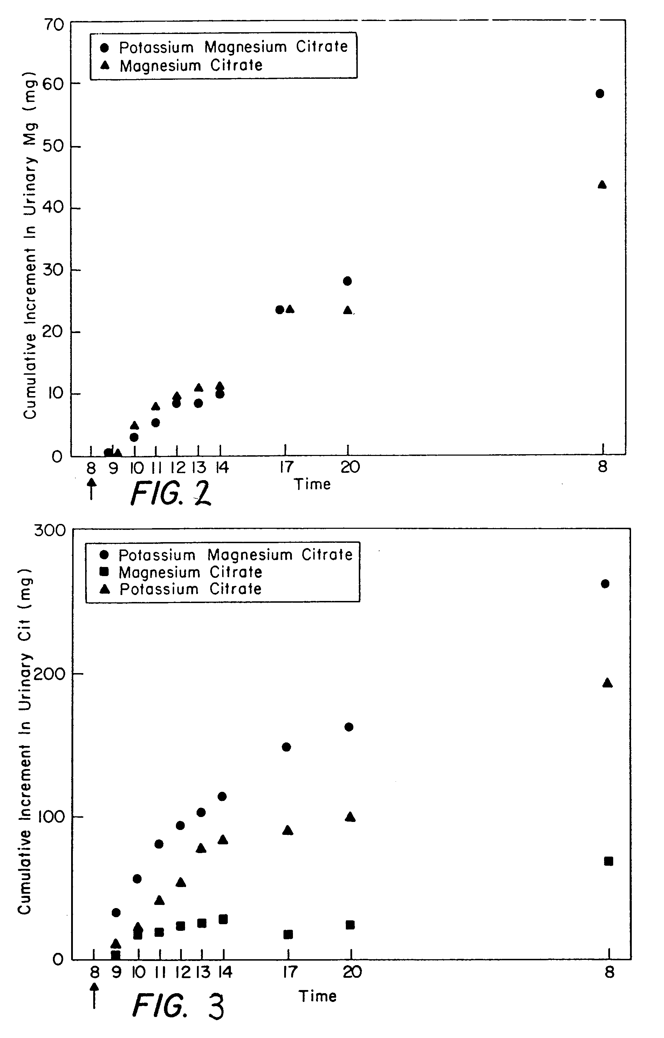

Magnesium bioavailability from potassium magnesium citrate was compared with that of magnesium citrate. At each time period (following oral administration), the difference in urinary magnesium following ingestion of potassium magnesium citrate (containing 24.5 meq magnesium) or magnesium citrate (containing 25 meq magnesium) from that obtained at a corresponding time period following taking potassium citrate (no magnesium, thus serving as control) was calculated. The cumulative increment in urinary magnesium (indicative of magnesium bioavailability or absorption) is shown in Figure 2. Note equivalent magnesium bioavailability between the two preparations.

Citrate bioavailability from potassium magnesium citrate was compared with that of potassium citrate and magnesium citrate. At each time period, the difference in urinary citrate following administration of potassium magnesium citrate (73.5 meq citrate), potassium citrate (50 meq citrate) or magnesium citrate (25 meq citrate) from that obtained following ingestion of potassium chloride (containing no citrate, thus serving as control) was calculated. The cumulative increment in citrate was much higher for potassium magnesium citrate than for the other two preparations, as shown in Figure 3.

### Example 2

A study was done to demonstrate the comparative physiological-physiochemical action of tetrapotassium monomagnesium dicitrate prepared when administered orally. The study was performed with two patients, and the results are detailed below:

Each patient underwent five phases of study: placebo, potassium chloride, potassium citrate, magnesium citrate, and potassium-magnesium citrate. The results (mean values for the two patients) are outlined in Table 1. Compared to potassium chloride, potassium magnesium citrate gave a higher urinary pH, magnesium, and citrate. Compared to potassium citrate, potassium magnesium citrate produced a greater citrate excretion as well as enhanced magnesium excretion. Compared to magnesium citrate, potassium magnesium citrate gave higher values for urinary pH and citrate. Finally, compared to the placebo, potassium magnesium citrate produced higher values for urinary pH, magnesium, potassium and citrate, and lower values for urinary calcium and oxalate.

**TABLE 1**

| Physiological Effects | | | | | |
|---|---|---|---|---|---|
| | Placebo | Potassium Chloride | Potassium Citrate | Magnesium Citrate | Potassium-Mg Citrate |
| No tablets/d | 7 | 7 | 10 | 2.5 | 7 |
| K content, meq/d | 0 | 49 | 50 | 0 | 49 |
| Mg content, meq/d | 0 | 0 | 0 | 25 | 24.5 |
| Citrate content, meq/d | 0 | 0 | 50 | 25 | 73.5 |

| Urinary | | | | | |
|---|---|---|---|---|---|
| pH | 6.26 | 5.84 | 6.93 | 6.13 | 6.94 |
| Ca,mg/d | 145 | 103 | 86 | 137 | 106 |
| Mg,mg/d | 64 | 73 | 69 | 113 | 116 |
| Na,meq/d | 95 | 81 | 91 | 82 | 92 |
| K,meq/d | 31 | 90 | 80 | 38 | 73 |
| Citrate, mg/d | 498 | 636 | 821 | 718 | 968 |
| Oxalate, mg/d | 27 | 27 | 27 | 23 | 23 |

It is expected that potassium magnesium citrate should be equally effective as potassium chloride in preventing thiazide-induced hypokalemia, except in rare patients with severe chloride deficiency. Potassium magnesium citrate might be more effective than potassium citrate in augmenting citrate excretion, due to the "citraturic action" of magnesium, and the higher content of citrate. Moreover, the provision of magnesium as potassium magnesium citrate should augment urinary magnesium (from absorbed magnesium) and reduce urinary oxalate (from binding of oxalate by magnesium in the intestinal tract). Potassium magnesium citrate should also cause a greater enhancement of citrate excretion than magnesium citrate, because of its greater citrate content. Finally, it is theoretically possible that alkali load from potassium magnesium citrate might cause a further reduction in calcium excretion. This action might oppose the modest calciuric action of magnesium. Potasium magnesium citrate should therefore be more effective than potassium citrate or magnesium citrate in lowering urinary saturation of calcium oxalate and in increasing its inhibitor activity.

## Claims

1. The use of potassium magnesium citrate in a single salt in the manufacture of an orally administrable medicament for increasing urinary citrate excretion.

2. The use of potassium magnesium citrate in a single salt in the manufacture of an orally administrable medicament for increasing urinary magnesium excretion.

3. The use of potassium magnesium citrate in a single salt in the manufacture of an orally administrable medicament for decreasing the urinary calcium of an individual.

4. The use of potassium magnesium citrate in a single salt in the manufacture of an orally administrable medicament for decreasing the urinary oxalate of an individual.

5. The use of any of claims 1 to 4 wherein the ratio of potassium ion to magnesium ion to citrate ion in said salt is about 4:1:2.

6. A product containing potassium magnesium citrate and a thiazide for their concomitant or sequential use in therapy.

7. A product as claimed in claim 6, wherein the potassium magnesium citrate is as further defined in claim 5.

## Patentansprüche

1. Verwendung von Kaliummagnesiumcitrat als Einzelsalz zur Herstellung eines oral verabreichbaren Arzneimittels zur Erhöhung der Citratausscheidung im Harn.

2. Verwendung von Kaliummagnesiumcitrat als Einzelsalz zur Herstellung eines oral verabreichbaren Arzneimittels zur Erhöhung der Magnesiumausscheidung im Harn.

3. Verwendung von Kaliummagnesiumcitrat als Einzelsalz zur Herstellung eines oral verabreichbaren Arzneimittels zum Absenken des Harncalciums eines Individuums.

4. Verwendung von Kaliummagnesiumcitrat als Einzelsalz zur Herstellung eines oral verabreichbaren Arzneimittels zum Absenken des Harnoxalats eines Individuums.

5. Verwendung nach einem der Ansprüche 1 bis 4, wobei das Verhältnis von Kaliumion zu Magnesiumion zu Citration in dem Salz 4 : 1 : 2 ist.

6. Erzeugnis, enthaltend Kaliummagnesiumcitrat und ein Thiazid für deren begleitende oder regelmäßige therapeutische Verwendung.

7. Erzeugnis nach Anspruch 6, bei dem das Kaliummagnesiumcitrat wie in Anspruch 5 definiert ist.

## Revendications

1. Utilisation de citrate de magnésium et de potassium dans un sel unique dans la fabrication d'un médicament administrable par voie orale pour augmenter l'excrétion de citrate par voie urinaire.

2. Utilisation de citrate de magnésium et de potassium dans un sel unique dans la fabrication d'un médicament administrable par voie orale pour augmenter l'excrétion de magnésium par voie urinaire.

3. Utilisation de citrate de potassium et de magnésium dans un sel unique dans la fabrication d'un médicament administrable par voie orale pour diminuer le calcium urinaire d'un individu.

4. Utilisation de citrate de magnésium et de potassium dans un sel unique dans la fabrication d'un médicament administrable par voie orale pour diminuer l'oxalate urinaire d'un individu.

5. Utilisation selon l'une quelconque des revendications 1 à 4, dans laquelle le rapport de l'ion potassium à l'ion magnésium à l'ion citrate dans ledit sel est d'environ 4:1:2.

6. Produit contenant un citrate de potassium et de magnésium et un thiazide pour leur utilisation concomitante ou séquentielle en thérapie.

7. Produit selon la revendication 6, dans lequel le citrate de potassium et de magnésium est de plus tel que défini selon la revendication 5.
